# EUROPEAN PATENT APPLICATION

(11) **EP 4 759 277 A1**
(43) Date of publication of application: **17.06.2026**
(21) Application number: 25213687.4
(22) Date of filing: 05.11.2025
(51) Int. Cl.: A61F 5/01

(54) **ANKLE FOOT ORTHOSIS**

(30) Priority: 06.11.2024 FR 2412166
(71) Applicant: Thuasne, 92300 Levallois Perret (FR)
(72) Inventor: Cooney, Timothy, London, SE22 8JN (GB)
(74) Representative: Wilding, Charlotte Katherine

(57) **Abstract**

The present invention relates to orthotic devices. In particular, the present invention relates to an ankle foot orthosis (AFO) per se, and to methods of fitting an AFO on a patient.

The ankle foot orthosis comprises a foot plate (1) with a non-trimmable portion (6) and at least one trimmable portion (7, 8); a calf abutment member (3) with a non-trimmable portion (4) and at least two trimmable portions (5, 6); and a strut (2) between the foot plate and the calf abutment member.

## Description

The present invention relates to orthotic devices. In particular, the present invention relates to an ankle foot orthosis (AFO) per se, and to methods of fitting an AFO on a patient.

The use of high strength and lightweight materials in the manufacture of orthotics can have clinical advantages. For instance, in studies of the lower limb, it has been demonstrated that lower weight ankle foot orthotics (AFOs) decrease energy expenditure in walking and can increase cadence in a patient. An AFO consists of a lower footplate, which supports the patient's foot, an elongate strut extending up and around the patient's ankle, and a cuff by which the AFO is secured to the patient's calf. It has been found that composite fibre materials can be effectively employed to offer an orthotic force pattern, *i.e.* the application of a force on pressure tolerant areas of a person's anatomy using an orthosis to force a biomechanical change, whilst simultaneously being lightweight and strong.

When 'fitting' a pre-fabricated AFO to a patient, a specialist medical practitioner or orthotist usually needs to trim areas of the AFO, especially around the peripheral edge of the footplate, as one size of pre-fabricated AFO will be required to fit a range of different foot/shoe sizes. However, trimming composite fibre materials is difficult, and requires a specialist cutting and sanding machine. For some orthotists who are mobile between hospital or business locations it is likely that the only tooling available to them would be household scissors, which would not be capable of trimming many types of orthotic composites. For this reason, an orthotist that does not have access to suitable trimming tooling/machinery may need to use AFOs comprising plastics, such as polypropylene or polyethylene, instead of composite fibres. These AFOs can be easily trimmed using scissors, thereby allowing the practitioner to fit the AFO for a given user. However, a plastic AFO may not be as lightweight and/or as strong as a composite fibre AFO, and so may provide the user with an inferior medical outcome compared to that when a composite fibre AFO is used.

The present invention arises from the inventors' work in trying to overcome the problems associated with the prior art.

Accordingly, in a first aspect of the invention, there is provided an ankle foot orthosis (AFO), comprising:
(i) a foot plate comprising a non-trimmable portion and at least one trimmable portion;
(ii) a calf abutment member comprising a non-trimmable portion and at least two trimmable portions, the at least two trimmable portions being located in an area at least adjacent to the top edge of the calf abutment member and the bottom edge of the calf abutment member; and
(iii) a strut extending between the footplate and the calf abutment member.

Advantageously, the claimed AFO can be cut on the top and bottom side of the calf cuff, enabling the AFO to be customized to fit a wide range of user leg shapes. For example, the height of the orthosis may be adjusted by cutting off a portion at the top edge if the patient has shorter legs. Alternatively, the height of the orthosis may be adjusted by cutting off a portion at the bottom edge if the patient has longer legs. Furthermore, the claimed product can be cut using simple office scissors. This is due to the fibers used, as the fineness of the fibers and the material used enable "cutability".

In one embodiment, the footplate is configured to extend beneath the sole of a foot of a subject. Typically, the footplate comprises a front which extends beneath the toes of the subject, a back which extends beneath the heel of the subject, and two sides which extend between the front and the back.

In one embodiment, the at least one trimmable portion of the foot plate is located in a hindfoot region of the footplate. In another embodiment, the at least one trimmable portion of the footplate is located in a forefoot region of the foot plate. In some embodiments, the footplate comprises at least two trimmable portions. Accordingly, the at least two trimmable portions of the foot plate may be located in a hindfoot region and a forefoot region of the foot plate.

In some embodiments, an area at least adjacent and/or towards the front of the footplate comprises the at least one trimmable portion. Accordingly, the at least one trimmable portion typically defines an extension to the length of the footplate.

In some embodiments, the at least one trimmable portion extends around the peripheral edge of the footplate at least adjacent to the position of the subject's toes when the orthosis is worn by a subject.

The trimmable portion towards the front of the footplate may increase the length of the footplate by at least 0.5 cm at its longest point, by at least 1.0 cm or 1.5 cm at its longest point, by at least 2.0 cm or 2.5 cm at its longest point, or by at least 3.0 cm at its longest point.

The trimmable portion towards the front of the footplate may increase the length of the footplate by between 0.5 cm and 10.0 cm at its longest point, by between 1.0 cm and 7.5 cm or between 1.5 cm and 5.0 cm at its longest point, or by between 2.0 cm and 4.0 cm or between 2.5 cm and 3.5 cm at its longest point.

Alternatively, or additionally, an area at least adjacent and/or towards a region of one or both sides of the footplate comprises the at least one trimmable portion. Typically, the area at least adjacent and/or towards a region of one or both sides of the footplate is also at least adjacent and/or towards the front of the footplate. Accordingly, the at least one trimmable portion preferably defines an extension to the width on at least one side of the footplate.

The trimmable portion along the width of the footplate may increase the width of the footplate by at least 0.25 cm at its widest point, by at least 0.5 cm or 1.0 cm at its widest point, or by at least 1.5 cm at its widest point.

The trimmable portion along the width of the footplate may increase the width of the footplate by between 0.25 cm and 10.0 cm at its widest point, by between 0.5 cm and 7.5 cm or between 1.0 cm and 5.0 cm at its widest point, or by between 1.5 cm and 2.5 cm at its widest point.

In some embodiments, the at least one trimmable portion defines an extension to both sides of the footplate.

Accordingly, the trimmable portion along the width of the footplate may increase the width of the footplate by at least 0.125 cm on each side at its widest point, by at least 0.25 cm or 0.5 cm on each side at its widest point, or by at least 0.75 cm on each side at its widest point.

The trimmable portion along the width of the footplate may increase the width of the footplate by between 0.125 cm and 5.0 cm on each side at its widest point, by between 0.25 cm and 3.75 cm or between 0.5 cm and 2.5 cm on each side at its widest point, or by between 0.75 cm and 1.25 cm on each side at its widest point.

In some embodiments, an area at least adjacent and/or towards the back of the footplate comprises the at least one trimmable portion. Accordingly, in some embodiments, the at least one trimmable portion extends around the peripheral edge of the footplate at least adjacent to the position of the subject's heel when the orthosis is worn by a subject. Typically, however, a portion towards the back of the footplate is comprised of a non-trimmable material, such that it can connect with the strut.

Accordingly, the at least one trimmable portion may extend around the peripheral edge of the footplate at least adjacent to the position of the subject's heel when the orthosis is worn by a subject.

The trimmable portion towards the back of the footplate may increase the length of the footplate by at least 0.5 cm at its longest point, by at least 1.0 cm or 1.5 cm at its longest point, by at least 2.0 cm or 2.5 cm at its longest point, or by at least 3.0 cm at its longest point.

The trimmable portion towards the back of the footplate may increase the length of the footplate by between 0.5 cm and 10.0 cm at its longest point, by between 1.0 cm and 7.5 cm or between 1.5 cm and 5.0 cm at its longest point, or by between 2.0 cm and 4.0 cm or between 2.5 cm and 3.5 cm at its longest point.

The footplate may comprise at least two trimmable portions. Accordingly, in some embodiments, an area adjacent to the front of the footplate and an area adjacent to a portion of both sides of the footplate comprises the trimmable portion.

The footplate may comprise at least three or four trimmable portions. Accordingly, in some embodiments, an area adjacent to the front of the footplate, an area adjacent to the back of the footplate, and an area adjacent to a portion of both sides of the footplate comprises the trimmable portion.

Advantageously, this allows the front, sides and back of the footplate to be trimmed such that the orthosis may be configured to fit a range of different shoe sizes.

In some embodiments, the at least one trimmable portion extends around the entire peripheral edge of the footplate. Typically, however, a portion towards the back of the footplate is comprised of a non-trimmable material, such that it can connect with the strut.

Additionally, the low Young's modulus of the trimmable portion is advantageous due to the large flexural range of movement that occurs in this area during walking.

In some embodiments where the plurality of non-trimmable fibres comprise unidirectional fibres, the unidirectional fibres are disposed in the footplate and aligned substantially parallel to the long axis of the foot of a subject. It may be understood that the long axis of the foot is a theoretical straight line between the middle of the heel through the second toe of the subject. Advantageously, the unidirectional fibres strengthen the footplate along the long axis.

It will be appreciated that the calf abutment member is for abutting the calf of a subject.

The calf abutment member comprises at least two trimmable portions located in an area at least adjacent to the top edge and the bottom edge of the calf abutment member. In other words, one of the at least two trimmable portions extends around a top edge of the calf abutment member, and one of the at least two trimmable portions extends around a bottom edge of the calf abutment member. Accordingly, the height of the orthosis may be adjusted by cutting off a portion at the top edge if the patient has shorter legs. Alternatively, the height of the orthosis may be adjusted by cutting off a portion at the bottom edge if the patient has longer legs.

The calf abutment member may comprise at least three or four trimmable portions. In some embodiments, the calf abutment member comprises a trimmable portion located in an area at least adjacent to the top edge, the bottom edge, and the two side edges of the calf abutment member. It will be appreciated, therefore, that the calf abutment member can be trimmed around its entire edge (except where the strut is located). Accordingly, in some embodiments, the at least two trimmable portions extend around the entire peripheral edge of the calf abutment member. Typically, however, a portion located in the centre of the calf abutment member is comprised of a non-trimmable material, such that it can connect with the strut.

It will be appreciated that an area at least adjacent to the top edge of the calf abutment member is the area located at the highest point of the calf abutment member that sits over the patient's knee. An area at least adjacent to the bottom edge of the calf abutment member is the area located at the lowest point of the calf abutment member when worn by the patient.

In one embodiment, the calf abutment member is configured to extend around the back of the subject's leg.

In another embodiment, the calf abutment member is configured to extend around the front of the subject's leg. In other words, the calf abutment member extends around the subject's shin. In this embodiment, the calf abutment member may be a tibia shell (or an anterior shell).

In some embodiments, the non-trimmable portion of the calf-abutment member is located between the at least two trimmable portions at the top and bottom edge of the calf abutment member. In other words, the non-trimmable portion of the calf-abutment member is located in the middle of the calf abutment member, between the two trimmable portions at the top and bottom edge of the calf abutment member.

Additionally, in some embodiments, a portion towards the bottom edge of the calf abutment member is comprised of a non-trimmable material, such that it can connect with the strut. Typically, this non-trimmable portion is located in the centre of the calf abutment member.

The trimmable portion at the top edge may increase the height of the calf abutment member by at least 0.5 cm at its longest point, by at least 1.0 cm or 1.5 cm at its longest point, or by at least 2.0 cm or 2.5 cm at its longest point. Alternatively, the trimmable portion at the top edge may increase the height of the calf abutment member by at least 2.0 or 2.5 cm at its longest point, by at least 3.0 cm or 3.5 cm at its longest point, by at least 4.0 cm or 4.5 cm at its longest point, or by at least 5.0 cm at its longest point.

The trimmable portion at the top edge may increase the height of the calf abutment member by between 0.5 cm and 10.0 cm at its longest point, by between 1.0 cm and 7.5 cm or between 1.5 cm and 5.0 cm at its longest point, or by between 2.0 cm and 4.0 cm or between 2.5 cm and 3.5 cm at its longest point.

The trimmable portion at the bottom edge may increase the height of the calf abutment member by at least 0.5 cm at its longest point, by at least 1.0 cm or 1.5 cm at its longest point, or by at least 2.0 cm or 2.5 cm at its longest point. Alternatively, the trimmable portion at the bottom edge may increase the height of the calf abutment member by at least 2.0 or 2.5 cm at its longest point, by at least 3.0 cm or 3.5 cm at its longest point, by at least 4.0 cm or 4.5 cm at its longest point, or by at least 5.0 cm at its longest point.

The trimmable portion at the bottom edge may increase the height of the calf abutment member by between 0.5 cm and 10.0 cm at its longest point, by between 1.0 cm and 7.5 cm or between 1.5 cm and 5.0 cm at its longest point, or by between 2.0 cm and 4.0 cm or between 2.5 cm and 3.5 cm at its longest point.

Accordingly, the trimmable portion at the top and bottom edge may increase the height of the calf abutment member by at least 1.0 cm at its longest point, by at least 1.5 cm or 2.0 cm at its longest point, or by at least 2.5 cm or 3.0 cm at its longest point. Alternatively, the trimmable portion at the top and bottom edge may increase the height of the calf abutment member by at least 3.5 cm at its longest point, by at least 4.0 or 4.5 cm at its longest point, by at least 5.0 cm or 5.5 cm at its longest point, or by at least 6.0 cm or 6.5 cm at its longest point. Alternatively, the trimmable portion at the top and bottom edge may increase the height of the calf abutment member by at least 7.0 or 7.5 cm at its longest point, by at least 8.0 cm or 8.5 cm at its longest point, by at least 9.0 cm or 9.5 cm at its longest point, or by at least 10.0 cm at its longest point.

The trimmable portion at the top and bottom edge may increase the height of the calf abutment member by between 1.0 cm and 20.0 cm at its longest point, by between 1.0 cm and 15.0 cm, by between 1.0 cm and 10.0 cm. Alternatively, trimmable portion at the top and bottom edge may increase the height of the calf abutment member by between 5.0 cm and 20.0 cm at its longest point, or by between 10.0 cm and 20.0 cm or between 15.0 cm and 20.0 cm at its longest point.

The trimmable portion at the two side edges may increase the width of the calf abutment member by at least 0.5 cm at its widest point, by at least 1.0 cm or 1.5 cm at its widest point, or by at least 2.0 cm or 2.5 cm at its widest point. Alternatively, the trimmable portion at the two side edges may increase the width of the calf abutment member by at least 2.0 or 2.5 cm at its widest point, by at least 3.0 cm or 3.5 cm at its widest point, by at least 4.0 cm or 4.5 cm at its widest point, or by at least 5.0 cm at its widest point.

The trimmable portion at the two side edges may increase the width of the calf abutment member by between 0.5 cm and 10.0 cm at its widest point, by between 1.0 cm and 7.5 cm or between 1.5 cm and 5.0 cm at its widest point, or by between 2.0 cm and 4.0 cm or between 2.5 cm and 3.5 cm at its widest point.

In one embodiment, the orthosis comprises a releasable fastener for fastening the calf abutment member to a leg of a subject. The releasable fastener may comprise a strap for connecting opposite ends of the calf abutment member. Thus, the calf abutment member and the strap may be configured to encircle the calf of the subject, and tightening the strap may fasten the orthosis. The strap may be held in position by means of a Velcro fastener or a hook fastener.

The strut extends between the footplate and the calf abutment member.

In one embodiment, the strut is connected to the footplate at a location which is configured to be posterior (*i.e.* behind) to the ankle axis of a subject wearing the orthosis. In one embodiment, the strut is connected to the calf abutment member at a location which is configured to be posterior (*i.e.* behind) to the knee of a subject wearing the orthosis.

In another embodiment, the strut is connected to the footplate at a location which is configured to be along one of the two sides of the footplate. In some embodiments, the strut is connected to the footplate at a location which is configured to be along the outer side of the footplate (i.e., when worn by the patient, the outer side of the footplate is the side which would be furthest away from the patient's other foot). In one embodiment, the strut is connected to the calf abutment member at a location which is configured to be anterior (*i.e.* in front) to the knee of a subject wearing the orthosis.

In one embodiment, the strut is connected to the non-trimmable portion of the footplate, and the non-trimmable portion of the calf-abutment member.

In one embodiment, the strut is configured to extend over the back of a leg of the subject. In another embodiment, the strut is configured to extend over the front of the leg of the subject. In another embodiment, the strut is configured to extend over the side and/or front of the leg of the subject.

In some embodiments, the strut comprises or consists of a non-trimmable material. Accordingly, in some embodiments, the strut comprises or consists of carbon fibres. In some embodiments, the strut comprises or consists of fibreglass, polyester, or any other technical fibres.

Advantageously, the trimmable portions of the footplate and the calf cuff are visually distinct from the non-trimmable portion, due to the presence of guidelines or grooves. It will be appreciated that the guidelines or grooves (also referred to as indents, or trim lines), enable the footplate and/or calf cuff to be trimmed and shaped. The guidelines or grooves may be grey or black in colour.

Accordingly, in one embodiment, the at least one trimmable portion of the footplate comprises guidelines or grooves. In another embodiment, the at least two trimmable portions of the calf abutment member comprise guidelines or grooves. In some embodiments, the trimmable portions of the footplate and the calf abutment member comprise guidelines or grooves.

In some embodiments, the guidelines/grooves are located around the external periphery of the footplate. In some embodiments, the guidelines/grooves are located at the periphery around the front, sides and rear of the footplate. In some embodiments, the guidelines/grooves are located along the top and bottom edges of the calf abutment member.

In some embodiments, the footplate and calf abutment member comprises at least one set of guidelines/grooves, at least two sets of guidelines/grooves, or at least three sets of guidelines/grooves. As such, the footplate and calf abutment member may be trimmed to several different sizes.

In one embodiment, the trimmable portion comprises or consists of polyester fibres. In one embodiment, the at least one trimmable portion of the foot plate comprises or consists of polyester fibres. In one embodiment, the at least two trimmable portions of the calf abutment member comprise or consist of polyester fibres. Advantageously, the trimmable portion can be easily cut with scissors, allowing the orthosis to be adjusted for an individual subject.

The polyester fibres may be high tenacity polyester fibres. In some embodiments, the polyester fibres may be polyolefin fibres.

In one embodiment, the polyester fibre has a weight of between 50 and 150 gsm, between 60 and 140 gsm, between 70 and 130 gsm, between 80 and 120 gsm, or between 90 and 110 gsm.

In another embodiment, the polyester fibre has a weight of between 90 and 150 gsm, between 90 and 140 gsm, between 90 and 130 gsm, between 90 and 120 gsm, or between 90 and 110 gsm. In another embodiment, the polyester fibre has a weight of between 50 and 110 gsm, between 60 and 110 gsm, between 70 and 110 gsm, between 80 and 110 gsm, or between 90 and 110 gsm. Typically, the polyester fibre has a weight of 100 gsm.

In some embodiments, the trimmable portion (i.e., the polyester fibres) comprise a polymer resin. In some embodiments, the polyester fibres are impregnated with a polymer resin.

In some embodiments, the trimmable portion comprises between 20% and 60%, between 25% and 55%, between 30% and 50%, or between 35% and 45% polymer resin.

In another embodiment, the trimmable portion comprises between 35% and 60%, between 35% and 35%, between 35% and 50%, or between 35% and 45% polymer resin. In another embodiment, the trimmable portion comprises between 20% and 45%, between 25% and 45%, between 30% and 45%, or between 35% and 45% polymer resin. Typically, the trimmable portion comprises 40% polymer resin.

The polymer resin may be epoxy resin.

In another embodiment, the polymer resin may be acrylic.

In one embodiment, the at least one trimmable portion of the footplate comprises at least one layer of polyester fibres. In another embodiment, the trimmable portion of the footplate comprises at least two layers of polyester fibres. In another embodiment, the trimmable portion of the footplate comprises at least three, four or five layers of polyester fibres. In another embodiment, the trimmable portion of the footplate comprises at least six layers of polyester fibres.

In some embodiments, the polyester fibres of the footplate have a weight of at least 50 gsm, at least 75 gsm, or at least 100 gsm. In some embodiments, the polyester fibres of the footplate have a weight of between 50 gsm and 150 gsm, between 60 gsm and 140 gsm, between 70 gsm and 130 gsm, between 80 gsm and 120 gsm, or between 90 gsm and 110 gsm. In some embodiments, the polyester fibres of the footplate have a weight of between 50 gsm and 140 gsm, between 50 gsm and 130 gsm, between 50 gsm and 120 gsm, between 50 gsm and 110 gsm, or between 50 gsm and 100 gsm.

In some embodiments, the polyester fibres of the footplate have a fibre orientation of between 0° and 50°, or between 10° and 50°, or between 20° and 50°, or between 30° and 50°, or between 40° and 50°. In another embodiment, the polyester fibres of the footplate have a fibre orientation of between 0° and 40°, between 0° and 30°, between 0° and 20° or between 0° and 10°. In some embodiments, the polyester fibres of the footplate have a fibre orientation of 45°, 30°, 15° or 0°.

In one embodiment, the at least two trimmable portions of the calf abutment member comprise at least one layer of polyester fibres. In another embodiment, the at least two trimmable portions of the calf abutment member comprises at least two layers of polyester fibres. In another embodiment, the at least two trimmable portions of the calf abutment member comprises at least three, four or five layers of polyester fibres.

In some embodiments, the polyester fibres of the calf abutment member have a weight of at least 50 gsm, at least 75 gsm, or at least 100 gsm. In some embodiments, the polyester fibres of the calf abutment member have a weight of between 50 gsm and 150 gsm, between 60 gsm and 140 gsm, between 70 gsm and 130 gsm, between 80 gsm and 120 gsm, or between 90 gsm and 110 gsm. In some embodiments, the polyester fibres of the calf abutment member have a weight of between 50 gsm and 140 gsm, between 50 gsm and 130 gsm, between 50 gsm and 120 gsm, between 50 gsm and 110 gsm, or between 50 gsm and 100 gsm.

In some embodiments, the polyester fibres of the calf abutment member have a fibre orientation of between 0° and 50°, or between 10° and 50°, or between 20° and 50°, or between 30° and 50°, or between 40° and 50°. In another embodiment, the polyester fibres of the calf abutment member have a fibre orientation of between 0° and 40°, between 0° and 30°, between 0° and 20° or between 0° and 10°. In some embodiments, the polyester fibres of the calf abutment member have a fibre orientation of 45°, 30°, 15° or 0°.

In one embodiment, the non-trimmable portion comprises or consists of carbon fibres. Advantageously, due to the carbon fibre reinforced polymer, the non-trimmable portion is strong and lightweight.

The non-trimmable portion may comprise unwoven fibres. The unwoven fibres may comprise unidirectional fibres. Additionally, or alternatively, the non-trimmable portion may comprise woven fibres. The woven fibres may comprise bidirectional fibres (e.g. twill fibres, plain weave fibres).

In one embodiment, the non-trimmable portion comprises both unwoven and woven fibres. In another embodiment, the non-trimmable portion comprises both unidirectional fibres and bidirectional fibres (e.g., twill fibres). In another embodiment, the non-trimmable portion comprises both unidirectional carbon fibres and bidirectional carbon fibres. In another embodiment, the non-trimmable portion comprises unidirectional carbon fibres sandwiched between bidirectional carbon fibres.

Advantageously, the unidirectional fibres strengthen the orthosis along the axis to which they are aligned. Additionally, the bidirectional fibres (e.g., twill fibres) provide the orthosis with transverse strength.

Accordingly, in one embodiment the carbon fibres may comprise bidirectional (e.g., twill) carbon fibres and/or unidirectional (UD) carbon fibres. In one embodiment the carbon fibres may comprise bidirectional (e.g., twill) carbon fibres and unidirectional (UD) carbon fibres.

In one embodiment, the bidirectional carbon fibre has a weight of between 100 and 300 gsm, between 120 and 280 gsm, or between 140 and 260 gsm. In another embodiment, the bidirectional carbon fibre has a weight of between 160 and 240 gsm, or between 180 and 220 gsm.

In another embodiment, the bidirectional carbon fibre has a weight of between 180 and 300 gsm, between 180 and 280 gsm, between 180 and 260 gsm, between 180 and 240 gsm, or between 180 and 220 gsm. In another embodiment, the bidirectional carbon fibre has a weight of between 100 and 220 gsm, between 120 and 220 gsm, between 140 and 220 gsm, between 160 and 220 gsm, or between 180 and 220 gsm. Typically, the bidirectional carbon fibre has a weight of 200 gsm.

In one embodiment, the unidirectional (UD) carbon fibre has a weight of between 150 and 1000 gsm, between 200 and 900 gsm, or between 300 and 850 gsm. In another embodiment, the unidirectional (UD) carbon fibre has a weight of between 400 and 800 gsm, between 420 and 780 gsm, between 440 and 760 gsm, between 460 and 740 gsm, or between 480 and 720 gsm. In another embodiment, the unidirectional (UD) carbon fibre has a weight of between 500 and 700 gsm, between 520 and 680 gsm, between 540 and 660 gsm, between 560 and 640 gsm, or between 580 and 620 gsm.

In another embodiment, the unidirectional (UD) carbon fibre has a weight of between 580 and 800 gsm, between 580 and 780 gsm, between 580 and 760 gsm, between 580 and 740 gsm, between 580 and 720 gsm, or between 580 and 700 gsm. In another embodiment, the unidirectional (UD) carbon fibre has a weight of between 580 and 680 gsm, between 580 and 660 gsm, between 580 and 660 gsm, between 580 and 640 gsm, or between 580 and 620 gsm.

In another embodiment, the unidirectional (UD) carbon fibre has a weight of between 150 and 620 gsm, between 200 and 620 gsm, between 250 and 620 gsm, between 300 and 620 gsm, or between 350 and 620 gsm. In another embodiment, the unidirectional (UD) carbon fibre has a weight of between 400 and 620 gsm, between 420 and 620 gsm, between 440 and 620 gsm, between 460 and 620 gsm, between 480 and 620 gsm, or between 500 and 620 gsm. In another embodiment, the unidirectional (UD) carbon fibre has a weight of between 520 and 620 gsm, between 540 and 620 gsm, between 560 and 620 gsm, or between 580 and 620 gsm. Typically, the unidirectional (UD) carbon fibre has a weight of 600 gsm.

In some embodiments, the non-trimmable portion (e.g., the carbon fibres) comprises a polymer resin. In some embodiments, the carbon fibres are impregnated with a polymer resin.

In some embodiments, the non-trimmable portion comprises between 20% and 60%, between 25% and 55%, between 30% and 50%, or between 35% and 45% polymer resin.

In another embodiment, the non-trimmable portion comprises between 35% and 60%, between 35% and 35%, between 35% and 50%, or between 35% and 45% polymer resin. In another embodiment, the non-trimmable portion comprises between 20% and 45%, between 25% and 45%, between 30% and 45%, or between 35% and 45% polymer resin. Typically, the non-trimmable portion comprises 40% polymer resin.

The polymer resin may be epoxy resin.

In another embodiment, the polymer resin may be acrylic.

In one embodiment, the non-trimmable portion of the footplate comprises carbon fibres. In one embodiment, the non-trimmable portion of the footplate comprises bidirectional carbon fibres (e.g., carbon twill). In one embodiment, the non-trimmable portion of the footplate comprises unidirectional carbon fibres (carbon UD). In one embodiment, the non-trimmable portion of the footplate comprises unidirectional and bidirectional carbon fibres (e.g., carbon twill and carbon UD). In another embodiment, the non-trimmable portion of the footplate comprises fibreglass. In another embodiment, the non-trimmable portion of the footplate comprises fibreglass and unidirectional fibreglass.

In some embodiments, the carbon fibre or fibreglass (e.g., carbon twill or carbon UD) has a fibre orientation of between 0° and 50°, or between 10° and 50°, or between 20° and 50°, or between 30° and 50°, or between 40° and 50°. In another embodiment, the carbon fibre or fibreglass has a fibre orientation of between 0° and 40°, between 0° and 30°, between 0° and 20° or between 0° and 10°. In some embodiments, the carbon fibre or fibreglass has a fibre orientation of 45°, 30°, 15° or 0°. This non-trimmable portion is placed at the middle of the calf band to make it more rigid.

In one embodiment, the non-trimmable portion of the calf abutment member comprises carbon fibres. In one embodiment, the non-trimmable portion of the calf abutment member comprises bidirectional carbon fibres (e.g., carbon twill). In one embodiment, the non-trimmable portion of the calf abutment member comprises carbon UD. In one embodiment, the non-trimmable portion of the calf abutment member comprises bidirectional and unidirectional carbon fibres (e.g., carbon twill and carbon UD). In another embodiment, the non-trimmable portion of the calf abutment member comprises fibreglass. In another embodiment, the non-trimmable portion of the calf abutment member comprises fibreglass and unidirectional fibreglass.

In some embodiments, the carbon fibre or fibreglass (e.g., carbon twill or carbon UD) has a fibre orientation of between 0° and 50°, or between 10° and 50°, or between 20° and 50°, or between 30° and 50°, or between 40° and 50°. In another embodiment, the carbon fibre or fibreglass has a fibre orientation of between 0° and 40°, between 0° and 30°, between 0° and 20° or between 0° and 10°. In some embodiments, the carbon fibre or fibreglass has a fibre orientation of 45°, 30°, 15° or 0°. This non-trimmable portion is placed at the middle of the calf band to make it more rigid.

It will be appreciated that the AFO according to the first aspect, will be fitted to and worn by a patient.

Accordingly, in a second aspect of the invention, there is provided a method for fitting an ankle foot orthosis (AFO) onto a patient, the method comprising:
(i) trimming a foot plate of the AFO to fit the patient, wherein the foot plate comprises a non-trimmable portion and at least one trimmable portion;
(ii) trimming a calf abutment member of the AFO to fit the patient, wherein the calf abutment member comprises a non-trimmable portion and at least two trimmable portions, the at least two trimmable portions being located in an area at least adjacent to the top edge of the calf abutment member and the bottom edge of the calf abutment member; and
(iii) securing the AFO to the patient with a releasable fastener.

It will be appreciated that the AFO used in the method according to the second aspect is as described in the first aspect.

Advantageously, the AFO can be cut on the top and bottom side of the calf cuff, enabling the AFO to be customized to fit a wide range of user leg shapes. For example, the height of the orthosis may be adjusted by cutting off a portion at the top edge if the patient has shorter legs. Accordingly, in one embodiment, the method comprises trimming the calf abutment member along the trimmable portion located in an area at least adjacent to the top edge of the calf abutment member.

Alternatively, the height of the orthosis may be adjusted by cutting off a portion at the bottom edge if the patient has longer legs. Accordingly, in one embodiment, the method comprises trimming the calf abutment member along the trimmable portion located in an area at least adjacent to the bottom edge of the calf abutment member.

In one embodiment, the method comprises trimming the footplate along the trimmable portion.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-
**Figure 1** shows one embodiment of an ankle foot orthosis (AFO) according to the invention, comprising a trimmable calf abutment member. The trimmable portions are located at the top and bottom edge of the calf abutment member.
**Figure 2** shows one embodiment of an AFO according to the invention, comprising a trimmable footplate. The trimmable portions of the footplate are located towards and around the front of the footplate (i.e., where the user's toes are positioned), and towards the back of the footplate (i.e., where the user's heel is positioned).
**Figure 3** shows one embodiment of an AFO according to the invention. The trimmable portions of the calf abutment member and the footplate, may comprises grooves (indents or trim lines) to help guide the trimming of the calf abutment member and footplate.
**Figure 4** illustrates one embodiment of a trimmable footplate. The footplate comprises a trimmable portion around the front of the footplate (i.e., where the user's toes are positioned), and towards the back of the footplate (i.e., where the user's heel is positioned).
**Figure 5** illustrates one embodiment of a trimmable calf abutment member. The calf abutment member comprises a non-trimmable portion, located between two trimmable portions, at the top and bottom edge of the calf abutment member.
**Figure 6** illustrates another embodiment of a trimmable calf abutment member. The calf abutment member comprises a trimmable portion in an area at least adjacent to the top edge, the bottom edge, and the two side edges of the calf abutment member.

### Examples

The inventors set out to manufacture an AFO that is adjustable by the fitter (person in charge of the choice and fitting of the product on the patient).

### Composites

The inventors used different composites for this product:

| **Fiber** | **Fiber weight** | **Resin** |
|---|---|---|
| Carbon Twill | 100-300 gsm | Epoxy resin |
| Carbon UD | 400-800 gsm | |
| High tenacity polyester | 50-150 gsm | |

### Cuttability

The inventors wanted the height of the product and the length of the footplate to be adjustable. For that, the inventors defined a layup with different materials to be cuttable.

### Trimmable AFO

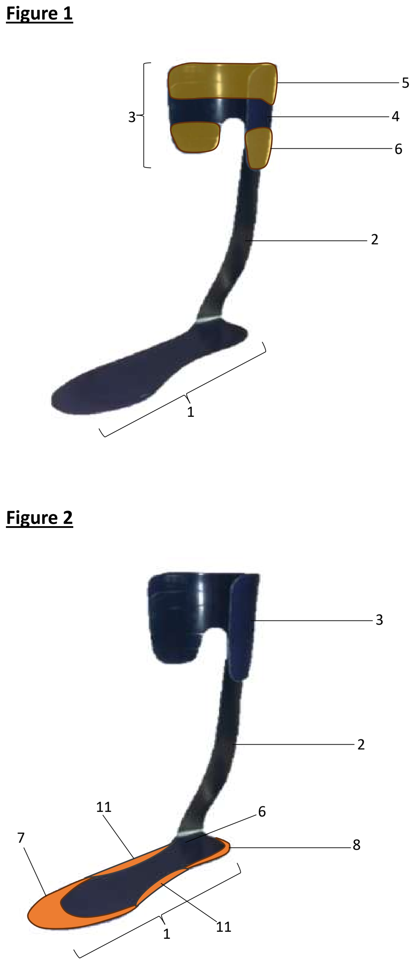

Figures 1 and 2 illustrate an exemplary embodiment of an AFO according to the invention, comprising a footplate 1, a strut 2, and a calf-abutment member 3.

As shown in Figure 1, the calf abutment member 3 comprises a non-trimmable portion 4 and at least two trimmable portions 5, 6. The trimmable portions 5, 6, are located on the top edge and bottom edge of the calf abutment member 3. The non-trimmable portion 4 is located between the trimmable portions 5, 6. Accordingly, the height of the orthosis may be adjusted by cutting off a portion at the top edge 5 if the patient has shorter legs. Alternatively, the height of the orthosis may be adjusted by cutting off a portion at the bottom edge 6 if the patient has longer legs.

As shown in Figure 2, the footplate 1, also comprises a non-trimmable portion 6, and at least one trimmable portion 7, 8. The trimmable potions 7, 8, are located in an area adjacent to the front of the footplate 7, an area adjacent to the back of the footplate 8, and an area adjacent to a portion of both sides of the footplate 11.

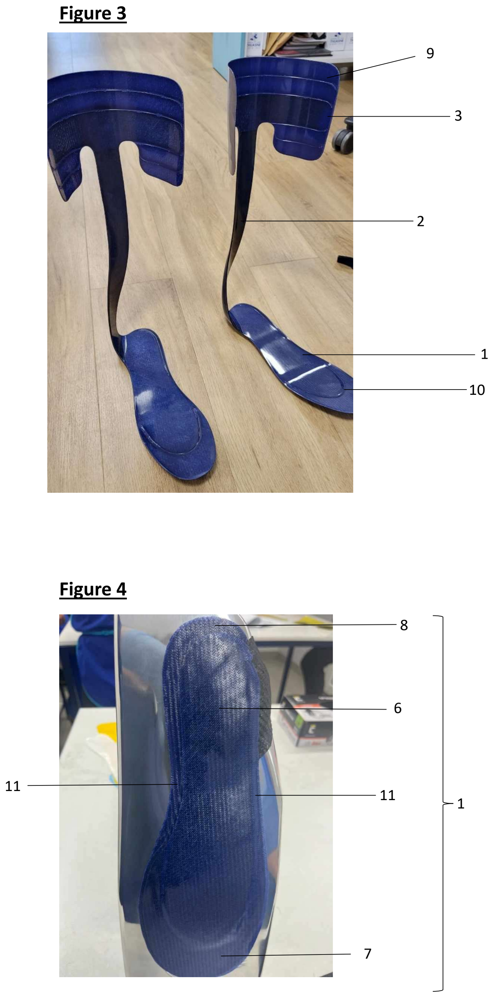

Figure 3 illustrates an exemplary embodiment of an AFO according to the invention, in which the trimmable portions of the footplate 1, and the calf abutment member 3, comprise grooves (indents/trim lines) 9, 10, to help guide the trimming of the calf abutment member and footplate. Accordingly, the wearer may determine the periphery that fits his or her feet and trim the footplate accordingly along the grooves in order to properly size the AFO.

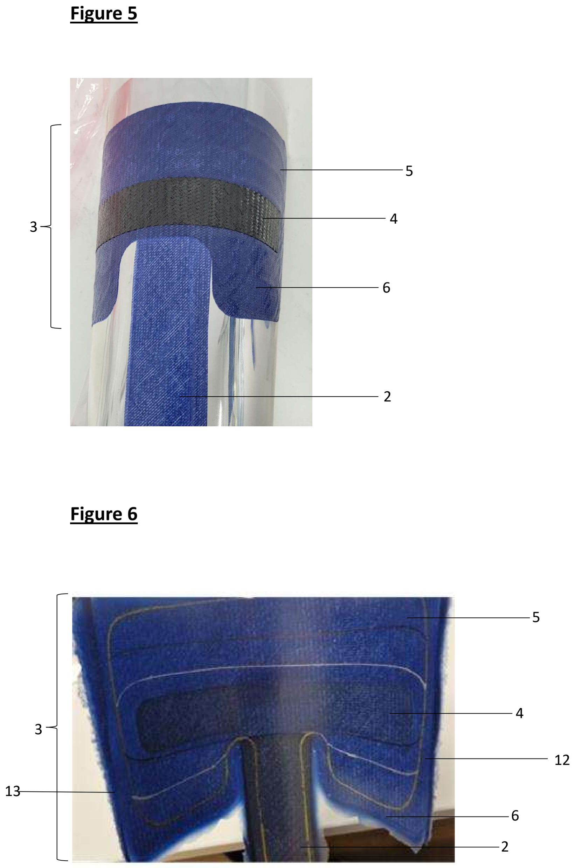

As shown in Figure 4, the footplate of the AFO comprises at least one trimmable portion around the front of the footplate 7 (i.e., where the user's toes are positioned), and towards the back of the footplate 8 (i.e., where the user's heel is positioned). Additionally, as shown in Figure 5, the calf abutment member of the AFO comprises a non-trimmable portion 4, located between two trimmable portions 5, 6, at the top and bottom edge of the calf abutment member. Additionally, as shown in Figure 6, the calf abutment member of the AFO may comprise two trimmable portions 5, 6, at the top and bottom edge of the calf abutment member, and two trimmable portions 12, 13, along the two side edges of the calf abutment member. The trimmable portions of the footplate and calf abutment member are typically comprised of polyester fibres, whereas the non-trimmable portions are typically comprised of carbon fibres.

## Claims

1. An ankle foot orthosis (AFO), comprising:
(i) a foot plate comprising a non-trimmable portion and at least one trimmable portion;
(ii) a calf abutment member comprising a non-trimmable portion and at least two trimmable portions, the at least two trimmable portions being located in an area at least adjacent to the top edge of the calf abutment member and the bottom edge of the calf abutment member; and
(iii) a strut extending between the footplate and the calf abutment member.

2. The AFO according to claim 1, wherein the at least one trimmable portion of the foot plate is located in a hindfoot region of the footplate, or wherein the at least one trimmable portion of the footplate is located in a forefoot region of the foot plate.

3. The AFO according claim 1, wherein the footplate comprises at least two trimmable portions, optionally wherein the at least two trimmable portions are located in a hindfoot region and a forefoot region of the foot plate.

4. The AFO according to claim 1, wherein an area at least adjacent and/or towards the front of the footplate comprises the at least one trimmable portion, optionally wherein the trimmable portion towards the front of the footplate increases the length of the footplate by at least 0.5 cm at its longest point, by at least 1.0 cm or 1.5 cm at its longest point, by at least 2.0 cm or 2.5 cm at its longest point, or by at least 3.0 cm at its longest point.

5. The AFO according to claim 1, wherein an area at least adjacent and/or towards a region of one or both sides of the footplate comprises the at least one trimmable portion, optionally wherein the trimmable portion along the width of the footplate increases the width of the footplate by at least 0.25 cm at its widest point, by at least 0.5 cm or 1.0 cm at its widest point, or by at least 1.5 cm at its widest point.

6. The AFO according to claim 1, wherein an area at least adjacent and/or towards the back of the footplate comprises the at least one trimmable portion, optionally wherein the trimmable portion towards the back of the footplate increases the length of the footplate by at least 0.5 cm at its longest point, by at least 1.0 cm or 1.5 cm at its longest point, by at least 2.0 cm or 2.5 cm at its longest point, or by at least 3.0 cm at its longest point.

7. The AFO according to claim 1, wherein the footplate comprises at least three or four trimmable portions, optionally wherein an area adjacent to the front of the footplate, an area adjacent to the back of the footplate, and an area adjacent to a portion of both sides of the footplate comprises the trimmable portion.

8. The AFO according to claim 1, wherein the at least one trimmable portion extends around the entire peripheral edge of the footplate.

9. The AFO according to claim 1, wherein the calf abutment member comprises a trimmable portion located in an area at least adjacent to the top edge, the bottom edge, and the two side edges of the calf abutment member.

10. The AFO according to claim 1, wherein the at least two trimmable portions extend around the entire peripheral edge of the calf abutment member.

11. The AFO according to claim 1, wherein the trimmable portion at the top edge increases the height of the calf abutment member by at least 0.5 cm at its longest point, by at least 1.0 cm or 1.5 cm at its longest point, or by at least 2.0 cm or 2.5 cm at its longest point, or wherein the trimmable portion at the top edge increases the height of the calf abutment member by at least 2.0 or 2.5 cm at its longest point, by at least 3.0 cm or 3.5 cm at its longest point, by at least 4.0 cm or 4.5 cm at its longest point, or by at least 5.0 cm at its longest point.

12. The AFO according to claim 1, wherein the trimmable portion at the bottom edge increases the height of the calf abutment member by at least 0.5 cm at its longest point, by at least 1.0 cm or 1.5 cm at its longest point, or by at least 2.0 cm or 2.5 cm at its longest point, or wherein the trimmable portion at the bottom edge increases the height of the calf abutment member by at least 2.0 or 2.5 cm at its longest point, by at least 3.0 cm or 3.5 cm at its longest point, by at least 4.0 cm or 4.5 cm at its longest point, or by at least 5.0 cm at its longest point.

13. The AFO according to claim 1, wherein the orthosis comprises a releasable fastener for fastening the calf abutment member to a leg of a subject.

14. The AFO according to claim 1, wherein the strut is connected to the non-trimmable portion of the footplate, and the non-trimmable portion of the calf-abutment member.

15. The AFO according to claim 1, wherein the strut comprises or consists of a non-trimmable material, optionally wherein the strut comprises or consists of carbon fibres, fibreglass, or polyester.

16. The AFO according to claim 1, wherein the trimmable portions of the footplate and the calf abutment member comprise guidelines or grooves, and/or wherein the trimmable portions comprise or consist of polyester fibres.

17. The AFO according to claim 1, wherein the trimmable portions comprise a polymer resin, optionally wherein the trimmable portions comprise between 20% and 60%, between 25% and 55%, between 30% and 50%, or between 35% and 45% polymer resin, optionally wherein the polymer resin is epoxy resin or acrylic.

18. The AFO according to claim 16, wherein the trimmable portion of the footplate comprises at least two layers of polyester fibres, and/or wherein the at least two trimmable portions of the calf abutment member comprise at least two layers of polyester fibres.

19. The AFO according to claim 1, wherein:
(i) the non-trimmable portion comprises or consists of carbon fibres;
(ii) the non-trimmable portion comprises unidirectional fibres and/or bidirectional fibres, optionally wherein the non-trimmable portion comprises bidirectional carbon fibres and unidirectional (UD) carbon fibres; and/or
(iii) the non-trimmable portion comprises a polymer resin, optionally wherein the non-trimmable portion comprises between 20% and 60%, between 25% and 55%, between 30% and 50%, or between 35% and 45% polymer resin, optionally wherein the polymer resin is epoxy resin or acrylic.

20. A method for fitting an ankle foot orthosis (AFO) onto a patient, the method comprising:
(i) trimming a foot plate of the AFO to fit the patient, wherein the foot plate comprises a non-trimmable portion and at least one trimmable portion;
(ii) trimming a calf abutment member of the AFO to fit the patient, wherein the calf abutment member comprises a non-trimmable portion and at least two trimmable portions, the at least two trimmable portions being located in an area at least adjacent to the top edge of the calf abutment member and the bottom edge of the calf abutment member; and
(iii) securing the AFO to the patient with a releasable fastener.
